# EUROPÄISCHE PATENTSCHRIFT

(11) **EP 0 625 025 B1**
(45) Veröffentlichungstag und Bekanntmachung des Hinweises auf die Patenterteilung: **16.04.1997**
(21) Anmeldenummer: 94900697.7
(22) Anmeldetag: 06.12.1993
(51) Int. Cl.: A61B 7/04

(54) **SENSOR UND EINRICHTUNG ZUM MESSEN DES BLUTDRUCKES**
SENSOR AND DEVICE FOR MEASURING BLOOD PRESSURE
DETECTEUR ET DISPOSITIF PERMETTANT DE MESURER LA TENSION ARTERIELLE

(30) Priorität: 05.12.1992 CH 3721/92
(43) Veröffentlichungstag der Anmeldung: 23.11.1994
(73) Patentinhaber: AVL Medical Instruments AG, 8207 Schaffhausen (CH)
(72) Erfinder: LÖTSCHER, Bernhard, CH-8450 Andelfingen (CH)
(74) Vertreter: Krause, Walter, Dr. Dipl.-Ing.
(86) Internationale Anmeldenummer: CH9300273
(87) Internationale Veröffentlichungsnummer: WO9413207

(56) Entgegenhaltungen:
- EP-A- 0 297 146
- FR-A- 2 643 811
- US-A- 4 409 983
- US-A- 4 443 730

## Beschreibung

### Technisches Gebiet der Erfindung

Die Erfindung betrifft einen Sensor und eine Einrichtung zum Messen des Blutdruckes. Der zum Anliegen an einem eine Arterie enthaltenden Bereich eines lebenden, menschlichen oder eventuell tierischen Körpers vorgesehene Sensor, die Einrichtung und das Verfahren sollen eine nicht invasive und beispielsweise kontinuierliche oder mindestens quasi-kontinuierliche, sich beispielsweise über mehrere Stunden oder Tage erstreckende Blutdruckmessung ermöglichen.

Der Blutdruck wird häufig mit Einrichtungen gemessen, die auf der Methode von Riva-Rocci basieren und eine hohle, deformierbare Manschette besitzen. Diese wird für eine Messung am Oberarm oder einem anderen Körperteil einer zu untersuchenden Person befestigt, mit Luft aufgepumpt und wieder entlüftet. Beim Entlüften wird aufgrund der vom Blut beim Durchströmen einer Arterie erzeugten Korotkoff-Töne oder durch eine oszillometrische Messmethode der systolische und der diastolische Blutdruck ermittelt. Die Methode von Riva-Rocci ist nicht für eine längerdauernde, quasi-kontinuierliche Blutdruckmessung geeignet, da ein abwechselndes Aufpumpen und Entlüften der Manschette für den Patienten unangenehm und wahrscheinlich sogar gesundheitsschädlich wäre.

Es sind auch Blutdruckmesseinrichtungen mit Sensoren bekannt, um mit Licht oder Ultraschall die Strömungsgeschwindigkeit des Blutes oder Verformungen der Arterienwände zu messen. Es sei hierzu zum Beispiel auf die EP-A-0 467 853 verwiesen. Derartige Sensoren sind jedoch relativ teuer und erfordern eine sehr genaue Positionierung.

Ferner ist aus der US-A-5 111 826 eine Blutdruckmesseinrichtung mit einem Sensor bekannt, der einen piezoelektrischen Kraftaufnehmer aufweist. Dieser kann für eine Messung mit Haltemitteln, nämlich mit einer hohlen Manschette an einem Finger einer Person befestigt und an den Finger angedrückt werden. Ein solcher Sensor hat jedoch den Nachteil, dass von aussen oder infolge von Bewegungen des Patienten auf den Kraftaufnehmer einwirkende Kräfte Messfehler verursachen können. Zudem sind piezoelektrische Messwandler normalerweise auch pyroelektrisch, so dass der Sensor empfindlich auf Temperaturänderung ist. Die ein Fluid enthaltende, den Finger beim ganzen Umfang mit Druck beaufschlagende Manschette kann zudem bei einer langdauernden Messung die Durchblutung stören und behindert auch den Gebrauch der Hand sehr stark. Im übrigen enthält ein Finger keine grosse Arterie, was die Messgenauigkeit ebenfalls beeinträchtigt.

Aus EP-A-0 297 146 ist eine Blutdruckmesseinrichtung mit den Merkmale gemäß Oberbegriff des vorliegenden Anspruchs 1 bekannt.

### Darstellung der Erfindung

Der Erfindung liegt die Aufgabe zugrunde, einen Sensor und eine Einrichtung zum Messen des Blutdruckes zu schaffen, die Nachteile der bekannten Sensoren und Einrichtungen beheben. Dabei wird angestrebt, die Messergebnisse möglichst unabhängig von äusseren Kräften, von durch Bewegungen von Patienten verursachten Kräften und von Temperaturänderungen zu machen.

Diese Aufgabe wird gemäss der Erfindung gelöst durch einen Sensor gemäss dem Anspruch 1 und durch eine Einrichtung gemäss dem Anspruch 5.

### Kurze Beschreibung der Zeichnung

Der Erfindungsgegenstand wird nachfolgend anhand in der Zeichnung dargestellter Ausführungsbeispiele erläutert. In der Zeichnung zeigt
die Fig. 1 eine Schrägansicht der Haltemittel und des von diesen gehaltenen Sensors einer Einrichtung zum Messen des Blutdruckes,
die Fig. 2 eine schematische Schrägansicht der drei piezoelektrischen Kraftaufnehmer des Sensors und ein Blockschaltbild der zur Einrichtung gehörenden, elektronischen Schaltungsmittel,
die Fig. 3 ein den zeitlichen Verlauf eines Mess-Signals und des Blutdrucks darstellendes Diagramm,
die Fig. 4 eine Schrägansicht einer anderen Einrichtung zum Messen des Blutdruckes mit einem an einem Arm befestigbaren Band, das einen Sensor sowie ein Gehäuse mit elektronischen Schaltungsmitteln hält, und
die Fig. 5 ein Blockschaltbild der Kraftaufnehmer und der elektronischen Schaltungsmittel der in der Figur 4 ersichtlichen Einrichtung.

### Bevorzugte Ausführungsbeispiele der Erfindung

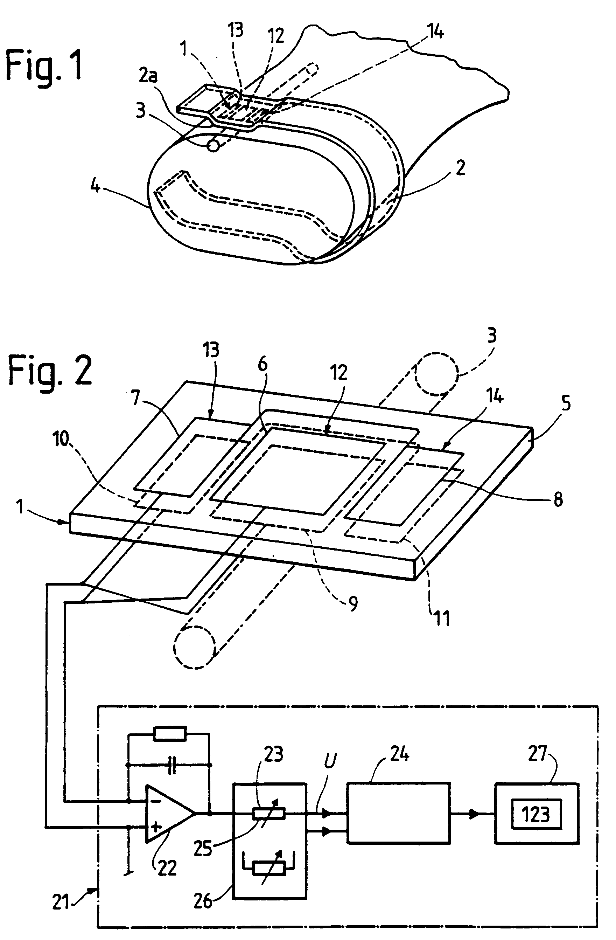

In der Fig. 1 ist ein Körperteil 4 eines zu untersuchenden Menschen, nämlich ein eine grosse Arterie 3 enthaltender Bereich eines Unterarms und eines Handgelenkes ersichtlich. Eine zum nicht-invasiven Messen des Blutdruckes dienende Einrichtung besitzt Sensormittel mit einem Sensor 1 und zum Halten und Andrücken der Sensors 1 an den Körperteil 4 dienende Haltemittel, die als Hauptbestandteil eine federnde, von einer Seite her wegnehmbar auf den Unterarm aufgesteckte, einen grossen Teil von dessen Umfang umschliessende, mehr oder weniger U-förmige Spange 2 besitzen. Diese hat bei dem sich im Querschnitt am nächsten bei der Arterie 3 befindenden Bereich der Oberfläche bzw. Haut des Körperteils 4 einen von den angrenzenden, mehr oder weniger geraden oder leicht gebogenen Spangenabschnitten weg nach innen, d.h. gegen den Körperteil 4 vorstehenden Vorsprung oder Höcker 2a. Der Sensor 1 ist an der dem Körperteil 4 zugewandten, mindestens annähernd ebenen Fläche des Höckers befestigt, beispielsweise mit einem Stück eines beidseitig klebenden Klebbandes lösbar angeklebt, das zum Beispiel weich und zusammendrückbar ist und kleine Deformationen und/oder Bewegungen des Sensors 1 ermöglicht. Eventuell kann zwischen dem Vorsprung oder Höcker 2a der Spange 2 und dem Sensor 1 noch ein elastisch zusammendrückbarer, eine Druckfederung bewirkender, zum Beispiel aus Schaumgummi bestehender Teil angeordnet sein, so dass sich der Sensor gut an die Haut anschmiegen und gut biegen sowie dehnen kann.

Der separat in der Fig. 2 gezeichneten Sensor 1 besitzt ein aus einem piezoelektrischen Material bestehenden Element, nämlich eine einstückige piezoelektrische Folie 5. Diese ist deformierbar, insbesondere biegbar, dehnbar sowie zusammendrückbar und besteht aus einem Kunststoff, nämlich aus Polyvinylidendifluorid (PVDF). Die beispielsweise viereck-, nämlich rechteckförmige Folie 5 ist auf ihrer sich in den Figuren 1 und 2 oben befindenden Fläche mit einer ersten, mittleren Mess-Elektrode 6 und auf einander abgewandten Seiten von dieser mit einer zweiten, bzw. dritten seitlichen Mess-Elektrode 7 bzw. 8 versehen. Auf der sich in den Figuren 1 und 2 unten befindenden Fläche der Folie 5 sind drei Gegen-Elektroden, nämlich eine erste, mittlere Gegen-Elektrode 9 und eine zweite sowie dritte, seitliche Gegen-Elektrode 10 bzw. 11 vorhanden. Die sechs Mess- und Gegen-Elektroden 6, 7, 8, 9, 10, 11 sind im wesentlichen quadrat- oder rechteckförmig und stehen sich paarweise gegenüber.

Die beiden ersten, mittleren Elektroden 6, 9 bilden zusammen mit dem sich zwischen ihnen befindenden Bereich der Folie 5 einen piezoelektrischen Messwandler, der als erster, mittlerer Kraftaufnehmer 12 dient. Die einander paarweise gegenüberstehenden zweiten, bzw. dritten Elektroden 7, 10 bzw. 8, 11 bilden zusammen mit den sich zwischen ihnen befindenden Bereichen der Folie 5 piezoelektrische Messwandler, die als zweiter, bzw. dritter, seitlicher Kraftaufnehmer 13 bzw. 14 dienen. Der zweite und der dritte Kraftaufnehmer 13 bzw. 14 befinden sich entsprechend der Anordnung der zu ihnen gehörenden Elektroden auf einander abgewandten Seiten des ersten, mittleren Kraftaufnehmers 12. Die verschiedenen Elektroden bestehen aus dünnen, metallischen Schichten, die durch Herausätzen aus den die beiden Flächen der Folie ursprünglich vollständig bedeckenden metallischen Schichten gebildet wurden. Die Oberfläche jeder zweiten und dritten Elektrode 7, 10 bzw. 8, 11 ist kleiner als die Oberfläche jeder ersten Elektrode 6, 9 und nämlich zum Beispiel ungefähr oder genau halb so gross wie die Oberfläche jeder ersten Elektrode 6, 9. Dementsprechend ist die bei einer Blutdruckmessung dem Körperteil 4 zugewandte Kraftaufnahmefläche des zweiten und des dritten Kraftaufnehmers 13 bzw. 14 kleiner und nämlich ungefähr oder genau halb so gross wie die Kraftaufnahmefläche des ersten Kraftaufnehmers 12

Die sich in der Fig. 2 oben befindende, erste Mess-Elektrode 6 ist elektrisch leitend mit der zweiten und dritten sich in der Fig. 2 unten befindenden Gegen-Elektrode 10 bzw. 11 verbunden. Die sich in der Fig. 2 unten befindende, erste Gegen-Elektrode 9 ist elektrisch leitend mit der zweiten und dritten, sich in der Fig. 2 oben befindenden Mess-Elektroden 7 bzw. 8 verbunden. Die für diese Verbindungen erforderlichen, elektrischen Leiter bestehen zum Beispiel aus Drähten, könnten aber auch zum Teil durch sich auf den beiden einander abgewandten Flächen der Folie 5 befindende Leiterbahnen gebildet sein. Die beiden seitlichen Kraftaufnehmer 13, 14 sind also elektrisch parallel zueinander und antiparallel zum ersten, mittleren Kraftaufnehmer 12 geschaltet.

Die piezoelektrische Folie 5 kann beispielsweise derart ausgebildet und polarisiert sein, dass zeitlich zunehmende, positive Kräfte bei den Mess-Elektroden 6, 7, 8 eine negative Ladung und bei den Gegen-Elektroden 9, 10, 11 eine positive Ladung erzeugen.

Die vom ersten, zweiten und dritten Kraftaufnehmer erzeugten, elektrischen Ladungen und/oder Spannungen werden im folgenden als erste bzw. zweite bzw. dritte Messgrösse bezeichnet. Jede dieser zeitlich variablen Messgrössen gibt ein Mass für die auf den betreffenden Kraftaufnehmer einwirkende, zeitlich ändernde Kraft und ist nämlich mindestens annähernd proportional zu dieser Kraft. Wenn die Ladung bzw. das Potential einer Mess-Elektrode bezüglich der ihr gegenüberstehenden Gegen-Elektrode negativ ist, wird der vom betreffenden Kraftaufnehmer erzeugten Messgrösse ein negatives Vorzeichen zugeordnet. Da der zweite und dritte Kraftaufnehmer parallel geschaltet sind, werden die zweite und dritte Messgrösse algebraisch addiert. Ferner wird die dabei entstehende Summe algebraisch von der ersten Messgrösse subtrahiert.

Die Einrichtung weist noch elektronische Schaltungsmittel 21 auf. Diese besitzen beispielsweise einen Ladungsverstärker 22, dessen invertierender Eingang mit den Elektroden 6, 10 und 11 verbunden ist. Der nicht-invertierende Eingang des Ladungsverstärkers 22 ist mit den Elektroden 7, 8, 9 verbunden. Der Ausgang des Ladungsverstärkers 22 ist über einen manuell einstellbaren Widerstand, der als Stellorgan 23 zum Einstellen der Empfindlichkeit dient, mit einer Auswertungsvorrichtung 24 verbunden. Das Stellorgan 23 bildet zusammen mit mindestens einem anderen, beispielsweise ebenfalls aus einem manuell einstellbaren Widerstand bestehenden Stellorgan 25 und beispielsweise weitern elektronischen Bauelementen Eichmittel 26, um mindestens einen Eichwert und/oder Eichparameter einzustellen und/oder um der Auswertungsvorrichtung 24 mindestens einen Eichwert und/oder Eichparameter in der Form eines elektrischen, analogen oder digitalen Signals einzugeben. Die Auswertungsvorrichtung 24 besitzt beispielsweise einen Analog/Digitalwandler zum Digitalisieren der vom Ladungsverstärker gelieferten Spannung und einen Prozessor zum digitalen Verarbeiten der digitalisierten Spannung. Die Auswertungsvorrichtung 24 hat einen Ausgang, der mit einer Anzeigevorrichtung 27 verbunden ist. Diese ist beispielsweise ausgebildet, um wahlweise einen mit dem systolischen oder diastolischen Blutdruck oder mit der Änderung des Blutdruckes verknüpften Relativ-Wert oder - nach einer vorherigen Eichung - direkt den Wert des systolischen und/oder diastolischen Blutdruckes in einer vorgegebenen oder wählbaren Druckeinheit beispielsweise digital anzuzeigen.

Der Sensor 1 wird für die Durchführung einer kontinuierlichen oder quasi-kontinuierlichen Blutdruckmessung mit Hilfe der die Spange 2 aufweisenden Haltemittel derart am Körperteil 4 angeordnet, dass der mittlere, erste Kraftaufnehmer 12 möglichst nahe bei der Arterie 3 an der Oberfläche des Körperteils 4 anliegt. Die beiden seitlichen Kraftaufnehmer 13 bzw. 14 sind ein wenig auf einander abgewandte Seiten der Arterie 3 versetzt und haben einen grösseren Abstand von dieser als der erste, mittlere Kraftaufnehmer 12.

Die elektronischen Schaltungsmittel 21 können beispielsweise in einem Gehäuse angeordnet sein, das mit irgendwelchen Haltemitteln am Körper des untersuchten Menschen gehalten ist. Falls dieser sich beispielsweise als Patient in einem Bett eines Spitals befindet, können die Schaltungsmittel 21 beispielsweise in einem gewissen Abstand vom Patienten angeordnet sein und über trennbare Steckverbindungsmittel mit dem Sensor 1 verbunden sein.

Beim Messen drückt die Spange 2 den Sensor 1 mit einer vorgegebenen Andruckkraft an die Oberfläche des Körperteils 4 an. Dieser übt eine die Andruckkraft kompensierende, von der Arterie 3 und vom Körperteil 4 weg nach aussen gerichtete Kraft auf die Kraftaufnehmer 12, 13 ,14 aus. Im statischen Zustand - d.h. wenn kein Blut durch die Arterie fliessen würde - verteilt sich die nach aussen gerichtete Kraft mindestens annähernd gleichmässig auf die ganze am Körperteil 4 anliegende Fläche des Sensor 1. Der vom Körperteil 4 auf die an diesem anliegenden Kraftaufnahmeflächen der Kraftaufnehmer 12, 13, 14 ausgeübte, statische Druck ist dann bei allen drei Kraftaufnehmern mindestens annähernd gleich gross.

Wenn nun das Herz pulsierend Blut durch die Arterie 3 pumpt und der Blutdruck in dem sich bei dem Sensor 1 befindenden Arterienabschnitt während eines gewissen Zeitintervalls vom diastolischen auf den systolischen Blutdruck ansteigt, breitet sich dieser Druckanstieg als Druckimpuls zur Oberfläche des Körperteils 4 aus. Da sich der erste, mittlere Kraftaufnehmer 12 näher bei der Arterie befindet als die beiden seitlichen Kraftaufnehmer 13, 14, wird die Druckzunahme beim ersten Kraftaufnehmer 12 grösser als beim zweiten und dritten Kraftaufnehmer 13 bzw. 14. Der Blutdruckanstieg hat also in Zusammenwirkung mit der von der Haltemitteln auf den Sensor ausgeübten Andruckkraft zur Folge, dass am ersten Kraftaufnehmer 12 zusätzlich zur statischen Kraft ein vom Druckimpuls verursachter, nach aussen gerichteter Druck-Kraftimpuls angreift. Dieser am ersten, mittleren Kraftaufnehmer 12 angreifende Kraftimpuls hat die Tendenz, den ganzen Sensor 1 nach aussen zu drücken, so dass die beiden zweiten Kraftaufnehmer 13, 14 entlastet werden. Ein Blutdruckanstieg erzeugt also beim ersten, mittleren Kraftaufnehmer 12 eine Druckerhöhung, d.h. eine Kraftänderung und bei den beiden seitlichen Kraftaufnehmern 13, 14 eine Verkleinerung der Druck-Kraft. Der Druckimpuls erzeugt dementsprechend eine Änderung der vom ersten Kraftaufnehmer 12 durch ein elektrisches Signal dargestellten, ersten Messgrösse und dieser Änderung entgegengesetzte Änderungen der vom dritten Kraftaufnehmer 13, 14 erzeugten zweiten bzw. dritten, durch ein elektrisches Signal dargestellten, zweiten bzw. dritten Messgrösse. Die Änderung der insgesamt von den Kraftaufnehmern abgegebenen elektrischen Ladung und/oder Spannung ist dann proportional zur Summe der Absolutwerte der von den drei Kraftaufnehmern gemessenen Kraftänderungen. Analoges gilt für die Kraftänderungen, die durch die Abnahme des Blutdruckes vom systolischen auf den diastolischen Blutdruckwert erzeugt werden.

Der Ladungsverstärker 22 erzeugt ein analoges elektrisches Signal, nämlich eine elektrische Spannung. Diese stellt eine vierte, zeitlich variable Messgrösse dar, die - abgesehen vom Vorzeichen - proportional zum Wert ist, der sich durch die beschriebene Verknüpfung der ersten, zweiten und dritten Messgrösse - d.h. durch Subtraktion der zweiten sowie der dritten Messgrösse von der ersten Messgrösse - ergibt. Die den vierten Messwert darstellende, elektrische Spannung ist entsprechend dem Blutdruck zeitlich ändernd. Die Auswertungsvorrichtung 24 kann dann die Maxima und Minima dieser Spannung ermitteln und diesen Werte zuordnen, die mit dem systolischen bzw. dem diastolischen Blutdruck verknüpft sind. Die Anzeigevorrichtung kann dann wahlweise den mit dem systolischen oder dem diastolischen Blutdruck verknüpfte Wert oder eventuell abwechselnd oder gleichzeitig beide Werte anzeigen. Der mit dem systolischen und/oder mit dem diastolischen Blutdruck verknüpfte Wert kann dann während längerer Zeit kontinuierlich oder quasi-kontinuierlich ermittelt und angezeigt werden.

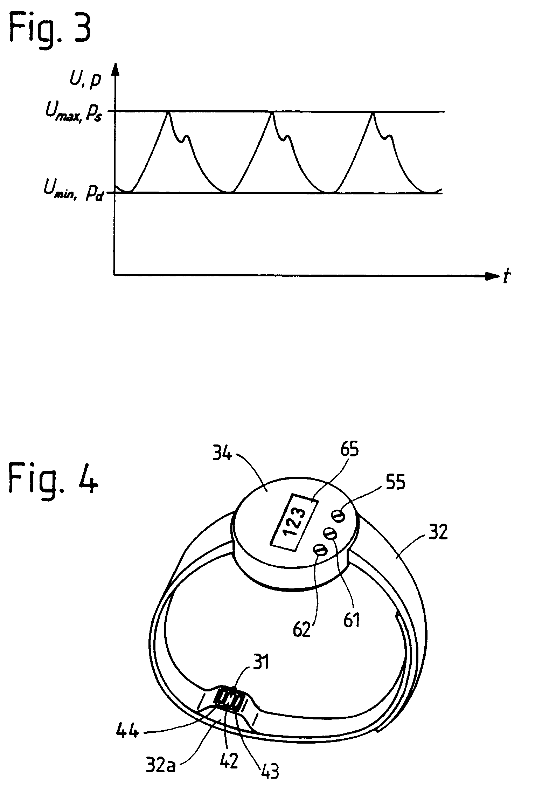

Man kann auch mit Hilfe einer zusätzlichen, nach der Methode von Riva-Rocci arbeitenden Eich-Messvorrichtung von Zeit zu Zeit eine Eich-Messung am untersuchten Menschen durchführen, indem an einem von dessen Arme vorübergehend eine aufblasbare, zur Eich-Messvorrichtung gehörende Manschette befestigt wird. Dann kann man mit der Auswertungsvorrichtung 24 mit Hilfe der Eichmittel 26 mindestens einen Eichwert und/oder Eichparameter einstellen und/oder in die Auswertungsvorrichtung 24 eingeben. Dies soll anhand der Fig. 3 erläutert werden. Diese zeigt ein Diagramm, auf dessen Abszisse die Zeit t und auf dessen Ordinate die zum vierten Messwert proportionale, der Auswertungsvorrichtung 24 zugeführte Spannung U sowie der Blutdruck p aufgetragen sind. Das Diagramm enthält ferner eine Kurve, die den zeitlichen Verlauf der Spannung U und des Blutdruckes p zeigt. Die Spannung U variiert zwischen einem Maximalwert Uₘₐₓ und einem Minimalwert Uₘᵢₙ. Nun wird angenommen, dass die Spannung U zunächst zwar mit dem Blutdruck verknüpft ist, dass aber die Anzeigevorrichtung 27 nur Relativ-Druckwerte anzeigt, die nicht direkt einen Blutdruckwert darstellen. Man kann nun mit der Eich-Messvorrichtung den systolischen Blutdruck pₛ sowie den diastolischen Blutdruck p_{d} messen und mit den Stellorganen 23, 25 die ungefähr gleichzeitig der Auswertungsvorrichtung 24 zugeführte Spannung U manuell derart einstellen, dass die Anzeigevorrichtung 27 den systolischen und den diastolischen Druck in vorgegebenen oder wählbaren Einheiten anzeigt.

Während der Benutzung der Einrichtung können zusätzlich zu der von den Haltemitteln erzeugten Andruckkraft und den vom Blutdruck erzeugten Kräfte eventuell aus irgend welchen Gründen vorübergehend noch andere, äussere und/oder durch Bewegungen des untersuchten Menschen verursachte Störkräfte auf den Sensor einwirken. Wenn eine solche Störkraft bei den Kraftaufnahmeflächen von allen drei Kraftaufnehmern 12, 13, 14 die gleiche Richtung hat sowie gleichmässig auf die ganzen Flächen der Folie 5 verteilt ist, werden die Kraftaufnahmeflächen der drei Kraftaufnehmer pro Flächeneinheit mit der gleichen Kraft und also im Fall einer Druckkraft mit dem gleichen Druck beaufschlagt. Die von der Störkraft verursachten Änderungen der von den drei Kraftaufnehmern erzeugten Messwerte kompensieren einander dann mindestens zu einem grossen Teil und vorzugsweise vollständig. Die Störkraft verändert daher die von den zusammengeschalteten Kraftaufnehmern insgesamt dem Ladungsverstärker 22 zugeführte Ladung und den durch diese dargestellten, vierten Messwert nicht, so dass die Störkraft auch die Blutdruckmessung nicht beeinflusst. Entsprechendes gilt für den Fall, dass die piezoelektrischen Kraftaufnehmer oder Messwandler 12, 13, 14 wegen einer Temperaturveränderung Ladungen oder Spannungen erzeugen und dadurch die drei ersten Messwerte ändern.

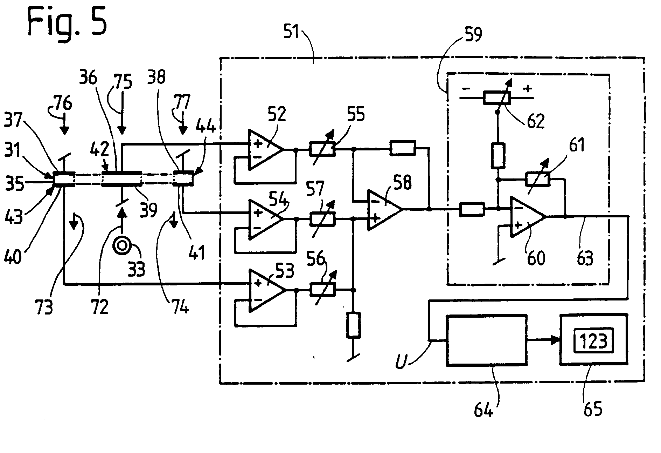

Die Figuren 4 und 5 zeigen die äussere Gestaltung und ein Blockschaltbild einer anderen zum Messen des Blutdruckes dienenden Einrichtung. Diese besitzt einen Sensor 31 und Haltemittel mit einem Band 32. Dieses besteht zum Beispiel aus zwei separaten Teilen, deren eine Enden an einem Gehäuse 34 befestigt sind und deren anderen Ende beispielsweise mit einem Klettverschluss oder mit irgend einem anderen Verschluss lösbar miteinander verbunden werden können. Das Band 32 ist derart ausgebildet, dass es den Unterarm der zu untersuchenden Person in der Nähe des Handgelenkes umschliessen kann, so dass diese Person die Blutdruckmesseinrichtung ähnlich wie eine Armbanduhr tragen kann. Das Band 32 besitzt einen Vorsprung oder Höcker 32a. Wenn das Band 32 am Arm befestigt ist, ragt der Vorsprung oder Höcker 32a nach innen gegen den Arm.

Der Sensor 31 ist an der bei der Benutzung dem Arm zugewandten Fläche des Vorsprungs oder Höckers 32a befestigt. Der Vorsprung oder Höcker 32a besteht mindestens zum Teil aus einem eine Druckfederung ergebenden Material, beispielsweise Schaumstoff. Der Vorsprung oder Höcker 32a ermöglicht, den Sensor 31 mit einer vorgesehenen Andruckkraft an den Arm anzudrücken, ohne dass das ganze Band 32 am Arm anliegt und ohne dass das Band übermässig straff gespannt werden muss, so dass das Band die Durchblutung des Arms nicht nennenswert behindert. Im übrigen ist der Sensor 31 und/oder mindestens ein Teil des Vorsprungs oder Höckers 32a vorzugsweise lösbar mit dem Band 32 verbunden, so dass der Sensor leicht ausgewechselt werden kann.

Der Sensor 31 besitzt eine in der Fig. 5 ersichtliche, einstückige, piezoelektrische Folie 35. Der mittlere Bereich der Folie 35 bildet zusammen mit einer Mess-Elektrode 36 sowie einer Gegen-Elektrode 39 einen ersten, mittleren Kraftaufnehmer 42. Die beiden seitlichen Bereiche der Folie 35 bilden zusammen mit einer zweiten und dritten Mess-Elektrode 37 bzw. 38 und einer zweiten und dritten Gegen-Elektrode 40 bzw. 41 einen zweiten und dritten, seitlichen Kraftaufnehmer 43 bzw. 44. Der Sensor 31 kann mit dem Band 32 derart am Arm befestigt werden, dass er in der Nähe einer grossen, in der Fig. 5 angedeuteten Arterie 33 an diesem anliegt.

In der Fig. 5 sind ferner elektronische Schaltungsmittel 51 ersichtlich. Diese sind im Gehäuse 34 untergebracht und besitzen drei Verstärker, die einen ersten Spannungsfolger 52, einen zweiten Spannungsfolger 53 und einen dritten Spannungenfolger 54 bilden. Der nicht-invertierende Eingang des ersten Spannungsfolgers 52 ist mit der Mess-Elektrode 36 des ersten Kraftaufnehmers 42 verbunden. Die nicht-invertierenden Eingänge des zweiten und dritten Spannnungsfolgers 53 bzw. 54 sind mit der Gegen-Elektrode 40 bzw. 41 des zweiten bzw. dritten Kraftaufnehmers 43 bzw. 44 verbunden. Die andern Elektroden 37, 38, 39 sind mit dem Massenanschluss der Schaltungsmittel verbunden.

Der Ausgang des ersten Spannungsfolgers 52 ist über einen als Stellorgan 55 dienenden, manuell einstellbaren Widerstand mit dem invertierenden Eingang eines Differentialverstärkers 58 verbunden. Die Ausgänge der beiden anderen Spannungsfolger 53, 54 sind über manuell einstellbare Widerstände, die als Stellorgan 56 bzw. 57 dienen, mit dem nicht-invertierenden Eingang des Differentialverstärkers 58 verbunden. Dessen Ausgang ist mit dem Eingang von Eichmitteln 59 verbunden, die einen Verstärker 60 und zwei manuell einstellbare Stellorgane 61 und 62 aufweisen, von denen das eine als Gegenkopplungswiderstand und das andere als Spannungsteilerwiderstand dient. Der Ausgang 63 des Verstärkers 60 ist über eine Auswertungsvorrichtung 64, die beispielsweise einen Analog/Digitalwandler und eventuell einen Prozessor aufweist, mit einer beispielsweise zur digitalen Anzeige ausgebildeten Anzeigevorrichtung 65 verbunden.

Beim Betrieb der in den Figuren 4 und 5 ersichtlichen Einrichtung wandeln die drei Kraftaufnehmer 42, 43, 44 die von ihnen aufgenommenen Kräfte in elektrische Mess-Signale, d.h. Ladungen und/oder Spannungen um. Die vom ersten, zweiten und dritten Kraftaufnehmer erzeugten, elektrischen Mess-Signale werden dann von den drei Spannungsfolgern 52, 53, 54 getrennt weiterverarbeitet. Die dem Differentialverstärker 58 von den drei Stellorganen 55, 56, 57 zugeführten, elektrischen Spannungen stellen dann die variable, erste, zweite und dritte Messgrösse dar. Die zeitlichen Änderungen dieser drei Messgrössen sind proportional zu den zeitlichen Änderungen der vom zugeordneten Kraftaufnehmer erzeugten Signale, wobei die Proportionalitätsfaktoren mit den Stellorganen 55, 56, 57 separat einstellbar sind. Der Differentialverstärker 58 subtrahiert dann die zweite und die dritte Messgrösse von der ersten Messgrösse. Die Stellorgane 55, 56, 57 ermöglichen, die Proportionalitätsfaktoren derart an individuelle Eigenschaften des Arms der untersuchten Person - beispielsweise an die Lage und Form der Arterie 33 und an das diese umgebende Gewebe - anzupassen, dass sich eine optimale möglichst vollständige Kompensation den von Störkräften bei den Kraftaufnehmern erzeugten Änderungen der Mess-Signale ergibt.

Die in den Figuren 4 und 5 ersichtliche Einrichtung kann am Anfang einer kontinuierlichen Blutdruckmessung geeicht werden. Wenn die Messung lange dauert, können nötigenfalls von Zeit zu Zeit neue Eichungen durchgeführt werden. Für eine Eichung kann man - wie es für die in den Figuren 1 und 2 gezeichnete Einrichtung beschrieben wurde - mit einer zusätzlichen Eich-Messvorrichtung den systolischen und den diastolischen Blutdruck messen und danach die der Auswertungsvorrichtung 64 zugeführte Spannung U aufgrund der mit der Eich-Messvorrichtung gemessenen Blutdruckwerte derart einstellen, dass die Anzeigevorrichtung 65 den systolischen und/oder diastolischen Blutdruck anzeigt. Dabei kann man mit dem einen Gegenkopplungswiderstand bildenden Stellorgan 61 die Differenz zwischen dem Maximalwert Uₘₐₓ und dem Minimalwert Uₘᵢₙ der Auswertungsvorrichtung 64 zugeführten Spannung U so einstellen, dass die Differenz zwischen den bei den Spannungen Uₘₐₓ und Uₘᵢₙ der angezeigten Druckwerte gleich der Differenz zwischen den mit der Eich-Messvorrichtung ermittelten Werten des systolischen Blutdruckes pₛ und des diastolischen Blutdruckes p_{d} ist. Danach kann man mit dem Stellorgan 62 den Nullpunkt so einstellen, dass die von der Anzeigevorrichtung 65 angezeigten Druckwerte dem systolischen bzw. diastolischen Blutdruck entsprechen.

In der Fig. 5 ist durch einen Pfeil die bei einem Anstieg des arteriellen Blutdrucks auf den ersten, mittleren Kraftaufnehmer 42 einwirkende pulsbedingte Kraftänderung 72 angedeutet. Des weiteren sind durch Pfeile die dann auf die seitlichen Kraftaufnehmer 43, 44 einwirkenden Entlastungs-Kraftänderungen 73, 74 dargestellt. Beim Messen werden die Absolutwerte der durch die pulsierende Blutströmung verursachten Kraftänderungen - entsprechend den Einstellungen der Stellorgane 55, 56, 57 miteinander verknüpft, nämlich gewichtet addiert.

In der Fig. 5 sind ferner von aussen auf die drei Kraftaufnehmer einwirkende Störkräfte 75, 76, 77 dargestellt. Wenn diese durch einen bei allen Kraftaufnehmern gleich grossen Druck erzeugt werden, ist die auf den mittleren Kraftaufnehmer 42 einwirkende Störkraft 75 - wegen der grösseren Kraftaufnahmeflache von diesem - grösser als die auf die beiden seitlichen Kraftaufnehmer einwirkenden Störkräfte 76, 77. Die Störkräfte werden dann bei der beim Differentialverstärker 58 stattfindenden Verknüpfung des ersten, zweiten und dritten Messwertes kompensiert.

Die Einrichtungen können noch in verschiedener Hinsicht geändert werden. Die drei Gegen-Elektroden der Sensoren 1 und 31 können zum Beispiel durch eine zusammenhängende, für alle drei Kraftaufnehmer des Sensors gemeinsame Gegen-Elektrode ersetzt werden. Die Sensoren 1 und 31 können ferner beispielsweise noch mit ihre Elektroden umschliessenden, aus metallischen Schichten bestehenden Abschirmungen versehen werden. Ferner kann man die mit Elektroden versehenen Flächen der Sensoren noch mit je zwei elektrisch isolierenden Schichten abdecken, zwischen denen eine elektrisch leitende Abschirmschicht angeordnet ist.

Die Eichmittel 26, 59 können ferner derart ausgebildet werden, dass sie elektrisch leitend mit der Eich-Messvorrichtung verbunden werden können. Diese kann dann zum Beispiel einen Prozessor aufweisen, um den Eichmitteln 26, 59 Eichwerte einzugeben und die elektronischen Schaltungsmittel 21, 51 automatisch zu eichen.

Im übrigen kann man Merkmale der beiden anhand der Figuren beschriebenen Einrichtungen miteinander kombinieren.

Des weitern können die zu den beiden seitlichen Kraftaufnehmern gehörenden Bereiche der piezoelektrischen Folie eventuell entgegengesetzt zu den zum ersten, mittleren Kraftaufnehmer gehörenden Folienbereich polarisiert sein. Stattdessen können die drei Kraftaufnehmer eventuell je ein separates piezolektrisches, aus einer Kunststoff-Folie oder eventuell aus keramischem oder kristallinem Material bestehendes Element besitzen, wobei die Elemente der beiden seitlichen Kraftaufnehmer dann entgegengesetzt zum Element des mittleren Kraftaufnehmers sein können. Ferner können die drei separaten Kraftaufnehmer dann an einem sich mechanisch miteinander verbindenden Verbindungselement befestigt sein. Bei diesen zwei Varianten sind die Folien oder sonstigen piezoelektrischen Elemente dann derart ausgebildet, dass gleich gerichtete Kraftänderungen bei den sich auf der gleichen Seite oder Fläche befindenden Elektroden elektrische Ladungen und Spannungen erzeugen, die beim ersten, mittleren Kraftaufnehmer ein anderes Vorzeichen haben als bei den beiden seitlichen Kraftaufnehmern.

Ferner kann jede der beschriebenen Blutdruck-Messeinrichtungen noch mit einem zweiten Sensor ausgerüstet werden, der gleich wie der beschriebene Sensor ausgebildet ist. Die beiden Sensoren können dann entlang der Arterie 3 bzw. 33 versetzt am Arm einer zu untersuchenden Person angeordnet werden. Die elektronischen Schaltungsmittel können dann ausgebildet sein, um aus der zeitlichen Verschiebung zwischen den von den beiden Sensoren gemessenen, zeitlich ändernden Messwerten die Pulswellengeschwindigkeit zu ermitteln und diese ebenfalls zur Bestimmung des Blutdrucks zu verwerten. Dies kann beispielsweise analog zu den in der bereits zitierten EP-A-0 467 853 beschriebenen Methoden erfolgen. Es sei hiermit ausdrücklich auf den Inhalt der EP-A-0 467 853 hingewiesen.

## Patentansprüche

1. Sensor zum Messen des Blutdruckes, mit Haltemitteln zum Andrücken eines ersten Kraftaufnehmers (12, 42) an einen eine Arterie (3, 33) enthaltenden Bereich eines lebenden Körpers, wobei zusätzlich zum ersten Kraftaufnehmer (12, 42) noch ein zweiter und ein dritter Kraftaufnehmer (13, 14, 43, 44) vorhanden und auf einander abgewandten Seiten des ersten Kraftaufnehmers (12, 42) angeordnet sind, **dadurch gekennzeichnet**, daß die drei Kraftaufnehmer (12, 13, 14, 42, 43, 44) je eine zum Anliegen am Körper bestimmte Kraftaufnahmefläche aufweisen, daß die Kraftaufnahmefläche des zweiten und des dritten Kraftaufnehmers (13, 14, 43, 44) kleiner ist als diejenige des ersten Kraftaufnehmers (12, 42), sowie daß der zweite und der dritte Kraftaufnehmer im Hinblick auf den ersten subtraktiv geschaltet ist.

2. Sensor nach Anspruch 1, **dadurch gekennzeichnet**, daß die drei Kraftaufnehmer (12, 13, 14, 42, 43, 44) als piezoelektrische Meßwandler ausgebildet sind.

3. Sensor nach Anspruch 1 oder 2, **dadurch gekennzeichnet**, daß die drei Kraftaufnehmer (12, 13, 14, 42, 43, 44) eine gemeinsame, flexible, aus piezoelektrischem Material bestehende Folie (5, 25) und auf dieser angeordnete Elektroden (6, 7, 8, 9, 10, 11, 36, 37, 38, 39, 40, 41) aufweisen.

4. Sensor nach einem der Ansprüche 1 bis 3, **dadurch gekennzeichnet**, daß die Haltemittel mindestens einen federnden Teil, beispielsweise eine Feder-Spange (2) und/oder beispielsweise einen Schaumstoffteil aufweisen, und die drei Kraftaufnehmer (12, 13, 14, 42, 43, 44) federnd an den lebenden Körper andrücken.

5. Einrichtung zum Messen des Blutdruckes, mit einem Sensor nach einem der Ansprüche 1 bis 4, wobei der Sensor ausgebildet ist, um eine erste Meßgröße, eine zweite Meßgröße und eine dritte Meßgröße zu bilden, die ein Maß für die vom ersten bzw. zweiten bzw. dritten Kraftaufnehmer gemessene Kraft und/oder Kraftänderung bilden, **dadurch gekennzeichnet**, daß der zweite und der dritte Kraftaufnehmer (13, 14, 43, 44) elektrisch parallel zueinander und antiparallel zum ersten Kraftaufnehmer (12, 42) geschaltet sind.

6. Einrichtung zum Messen des Blutdruckes, mit einem Sensor nach einem der Ansprüche 1 bis 4 und mit elektronischen Schaltungsmitteln, wobei die Schaltungsmittel ausgebildet sind, um eine erste Meßgröße, eine zweite Meßgröße und eine dritte Meßgröße zu bilden, die ein Maß für die vom ersten bzw. zweiten bzw. dritten Kraftaufnehmer gemessene Kraft und/oder Kraftänderung bilden, **dadurch gekennzeichnet**, daß die elektronischen Schaltungsmittel (51) mindestens ein manuell einstellbares Stellorgan (55, 56, 57) aufweisen, um von der ersten, zweiten und dritten Meßgröße mindestens eine einstellbar mit einem vom zugeordneten Kraftaufnehmer (12, 13, 14, 42, 43, 44) erzeugten, elektrischen Signal zu verknüpfen.

7. Einrichtung nach Anspruch 5 oder 6 **dadurch gekennzeichnet**, daß die Kraftaufnehmer (12, 13, 14, 42, 43, 44) und Schaltungsmittel (21, 51) ausgebildet sind, um die drei Meßgrößen derart zu verknüpfen, daß die Absolutwerte der Änderungen der zweiten und der dritten Meßgröße vom Absolutwert der Änderung der ersten Meßgröße subtrahiert werden.

8. Einrichtung nach Anspruch 5 oder 6, **dadurch gekennzeichnet**, daß die Kraftaufnehmer (12, 13, 14, 42, 43, 44) und Schaltungsmittel (21, 51) ausgebildet sind, um die zweite Meßgröße sowie die dritte Meßgröße von der ersten Meßgröße zu subtrahieren und dadurch eine ein Maß für den Blutdruck gebende, vierte Meßgröße zu bilden.

9. Einrichtung nach einem der Ansprüche 5 bis 8, **dadurch gekennzeichnet**, daß mindestens ein Teil der elektronischen Schaltungsmittel (51) in einem von den Haltemitteln gehaltenen Gehäuse (34) untergebracht ist.

10. Einrichtung nach einem der Ansprüche 5 bis 9, **dadurch gekennzeichnet**, daß die elektronischen Schaltungsmittel (21, 51) Eichmittel (25, 59) zum Einstellen und/oder Eingeben mindestens eines Eichwertes aufweisen und/oder mit solchen verbindbar und ausgebildet sind, um nach dem Einstellen und/oder Eingeben mindestens eines Eichwertes, der durch eine am Körper durchgeführte Messung mit einer Eich-Meßvorrichtung ermittelt wurde, den systolischen und/oder diastolischen Blutdruck zumindest quasi-kontinuierlich zu ermitteln.

## Claims

1. A sensor for measuring blood pressure, with fastening means for pressing a first force pick-up (12, 42) against a portion of a living body containing an artery (3, 33), where in addition to said first force pick-up (12, 42) a second and a third force pick-up (13, 14, 43, 44) are provided, which are located one on either side of said first force pick-up (12, 42), **characterized in that** the three force pick-ups (12, 13, 14, 42, 43, 44) each have a pick-up surface lying close to the body, and that the pick-up surface of the second and third force pick-up (13, 14, 43, 44) is smaller than that of the first force pick-up (12, 42), and further that the second and third force pick-ups are connected subtractively relative to the first force pick-up.

2. A sensor as in claim 1, **characterized in that** the three force pick-ups (12, 13, 14, 42, 43, 44) are configured as piezoelectric transducers.

3. A sensor as in claim 1 or 2, **characterized in that** the three force pick-ups (12, 13, 14, 42, 43, 44) are provided with one common piece of flexible foil (5, 25), which is made of piezoelectric material and carries a number of electrodes (6, 7, 8, 9, 10, 11, 36, 37, 38, 39, 40, 41).

4. A sensor as in any of claims 1 to 3, **characterized in that** the fastening means are provided with at least one spring component, for example, a spring clip (2), and/or a part made of foam rubber, elastically pressing the three force pick-ups (12, 13, 14, 42, 43, 44) against the living body.

5. A device for measuring blood pressure, with a sensor as described in any of claims 1 to 4, where said sensor is designed to form a first measurement variable and a second measurement variable and a third measurement variable, which measure the force and/or change of force obtained from the first and second and third force pick-up, **characterized in that** the second and third force pick-up (13, 14, 43, 44) are connected electrically parallel to each other and anti-parallel to the first force pick-up (12, 42).

6. A device for measuring blood pressure, with a sensor as described in any of claims 1 to 4, and with electronic circuitry, where said circuitry is designed to form a first measurement variable and a second measurement variable and a third measurement variable measuring the force and/or change of force obtained from the first and second and third force pick-up, **characterized in that** the electronic circuitry (51) is provided with at least one manually adjustable adjusting element (55, 56, 57) in order to combine adjustably at least one of the first, second and third measurement variables with an electric signal generated by the respective force pick-up (12, 13, 14, 42, 43, 44).

7. A device as in claim 5 or 6, **characterized in that** the force pick-ups (12, 13, 14, 42, 43, 44) and the circuitry (21, 51) are designed to combine the three measurement variables in such a way that the absolute values of the changes in the second and third measurement variable are subtracted from the absolute value of the change in the first measurement variable.

8. A device as in claim 5 or 6, **characterized in that** the force pick-ups (12, 13, 14, 42, 43, 44) and the circuitry (21, 51) are designed to subtract the second measurement variable and the third measurement variable from the first measurement variable, thus forming a fourth measurement variable as a measure for the blood pressure.

9. A device as in any of claims 5 to 8, **characterized in that** at least part of the electronic circuitry (51) is located in a housing (34) supported by the fastening means.

10. A device as in any of claims 5 to 9, **characterized in that** the electronic circuitry (21, 51) is provided with calibration means (25, 59) for setting and/or entering at least one calibration value, and/or can be connected thereto, permitting determination of the systolic and/or diastolic blood pressure in at least quasi-continuous fashion after at least one calibration value has been set and/or entered, this value having been obtained by a measurement on the body with a calibration/measuring device.

## Revendications

1. Senseur destiné à mesurer la pression sanguine, avec des moyens de fixation pour presser un premier capteur de force (12, 42) contre une région d'un corps vivant contenant une artère (3, 33), un deuxième et un troisième capteur de force (13, 14, 43, 44) s'ajoutant au premier capteur de force (12, 42) et étant agencés sur les faces opposées du premier capteur de force (12, 42), **caractérisé en ce que** chacun des trois capteurs de force (12, 13, 14, 42, 43, 44) possède une surface de captage de force destinée à s'appuyer contre le corps, en ce que la surface de captage de force du deuxième et du troisième capteur de force (13, 14, 43, 44) est inférieure à celle du premier capteur de force (12, 42), et en ce que le deuxième et le troisième capteur de force sont connectés de manière soustractive par rapport au premier.

2. Senseur selon la revendication 1, **caractérisé en ce que** les trois capteurs de force (12, 13, 14, 42, 43, 44) sont conçus comme transformateurs piézoélectriques de mesure.

3. Senseur selon la revendication 1 ou 2, **caractérisé en ce que** les trois capteurs de force (12, 13, 14, 42, 43, 44) comportent une feuille (5, 25) commune, flexible, réalisée en un matériel piézoélectrique, sur laquelle sont disposés des électrodes (6, 7, 8, 9, 10, 11, 36, 37, 38, 39, 40, 41).

4. Senseur selon l'une des revendications 1 à 3, **caractérisé en ce que** les moyens de fixation comportent au moins une pièce élastique, par exemple, une agrafe à ressort (2) et/ou, par exemple, une partie en mousse, et pressent les trois capteurs de force (12, 13, 14, 42, 43, 44) contre le corps vivant d'une manière élastique.

5. Dispositif de mesure de la pression sanguine, avec un senseur selon l'une des revendications 1 à 4, le senseur étant conçu de manière à former une première grandeur de mesure, une deuxième grandeur de mesure et une troisième grandeur de mesure qui forment une mesure pour la force mesurée et/ou le changement de force mesuré par le premier respectivement le deuxième respectivement le troisième capteur de force, **caractérisé en ce que** le deuxième et le troisième capteur de force (13, 14, 43, 44) sont électriquement connectés en parallèle l'un par rapport à l'autre et en antiparallèle par rapport au premier capteur de force (12, 42).

6. Dispositif de mesure de la pression sanguine, avec un senseur selon l'une des revendications 1 à 4 et avec des moyens électroniques de commutation, les moyens de commutation étant conçus de manière à former une première grandeur de mesure, une deuxième grandeur de mesure et une troisième grandeur de mesure qui forment une mesure pour la force mesurée et/ou le changement de force mesuré par le premier respectivement le deuxième respectivement le troisième capteur de force, **caractérisé en ce que** les moyens électroniques de commutation (51) comportent au moins un organe de réglage (55, 56, 57) réglable à la main, afin d'enchaîner de façon réglable au moins une de la première, deuxième et troisième grandeur de mesure avec un signal électrique généré par le capteur de force (12, 13, 14, 42, 43, 44) attribué.

7. Dispositif selon la revendication 5 ou 6, **caractérisé en ce que** les capteurs de force (12, 13, 14, 42, 43, 44) et les moyens de commutation (21, 51) sont conçus de manière à enchaîner les trois grandeurs de mesure de façon que les valeurs absolues des changements de la deuxième et de la troisième grandeur de mesure soient soustraits de la valeur absolue du changement de la première grandeur de mesure.

8. Dispositif selon la revendication 5 ou 6, **caractérisé en ce que** les capteurs de force (12, 13, 14, 42, 43, 44) et les moyens de commutation (21, 51) sont conçus de manière à soustraire la deuxième grandeur de mesure ainsi que la troisième grandeur de mesure de la première grandeur de mesure afin de former ainsi une quatrième grandeur de mesure qui donne une mesure pour la pression sanguine.

9. Dispositif selon l'une des revendications 5 à 8, **caractérisé en ce qu'**au moins une partie des moyens électroniques de commutation (51) est placée dans un boîtier (34) porté par les moyens de fixation.

10. Dispositif selon l'une des revendications 5 à 9, **caractérisé en ce que** les moyens électroniques de commutation (21, 51) comportent des moyens d'étalonnage (25, 59) destinés au réglage et/ou à l'introduction d'au moins une valeur étalonnée, et/ou peuvent être connectés avec de tels moyens d'étalonnage et sont conçus de manière à déterminer quasi-continuellement la pression sanguine systolique et/ou diastolique après le réglage et/ou l'introduction d'au moins une valeur étalonnée, déterminée par une mesure effectuée sur le corps par l'intermédiaire d'un instrument de mesure et d'étalonnage.
